# EUROPEAN PATENT APPLICATION

(11) **EP 0 634 178 A1**
(43) Date of publication of application: **18.01.1995**
(21) Application number: 94500122.0
(22) Date of filing: 11.07.1994
(51) Int. Cl.: A61L 11/00, A61L 2/18

(54) **Apparatus for the treatment of liquid hospital waste**

(30) Priority: 12.07.1993 ES 9301552
(71) Applicant: CARBONELL CIA. ANMA., S.A., E-08010 Barcelona (ES)
(72) Inventor: Jove Sanz, Ramon, E-08029 Barcelona (ES)
(74) Representative: Gomez-Acebo y Pombo, José Miguel

(57) **Abstract**

An apparatus for the treatment of liquid hospital waste, comprising a first (1) and a second (2) watertight treatment chamber, connected together by means of a syphon (9) and an overflow pipe (10), and provided with stirrers (14 and 15) for the waste. The first chamber is provided with an inlet (7-8) for receiving the waste, means for cutting off the entrance of the waste and causing it to be transferred to the second chamber (2), and means (21) for supplying a dispersing agent. The second chamber is provided with an outlet (11), means (20) for expelling the waste, and means for supplying a disinfecting agent. The different phases or stages are controlled by detectors comprising pressurestats.

## Description

The present invention relates to an apparatus for the treatment of liquid hospital waste with which it is possible to carry out the continuous treatment of said waste, as a result of which it reaches a state in which it is fit to be poured into the general sewage system.

### BACKGROUND OF THE INVENTION

The patent US 5.087.420 describes an apparatus for the treatment of infectious waste comprising a waste pulper, a tank for mixing the waste with a disinfectant and a recirculation and discharge pump. The different stages are controlled by means of a level sensor and a clock. This kind of apparatus is suitable for the treatment of solid waste, is high in cost and furthermore does not provide sufficient treatment of the waste such that it can be poured directly into the public sewage system.

The British patent 2.251.403 describes an apparatus for the treatment of waste and which makes it possible to simultaneously collect waste from one or several different points. This apparatus is provided with two containing chambers, each of which has a suction or vacuuming connector. Both containers are positioned at approximately the same height and their upper and lower parts are connected together by pipes of different cross sections. Furthermore, it includes another group of pipes and auxiliary control elements. This type of apparatus has an excessively complicated in terms of both its constitution and its operation.

### DESCRIPTION OF THE INVENTION

The object of the present invention is an apparatus for the treatment of liquid hospital waste which has a simple constitution and operation and a relatively low cost.

Another object of the invention is an apparatus by means of which the waste reaches a state in which it is fit to be poured into the town sewage system. To achieve this, the apparatus of the invention is constituted in such a way that it automatically carries out waste treatment in two consecutive stages as a result of which the waste that is treated fulfils the required hygienic and ecological conditions.

According to the present invention, the apparatus for the treatment of liquid hospital waste consists of a first and a second watertight treatment chamber, connected together by means of a syphon and an overflow pipe, both of said chambers being provided with stirrers for the liquid waste.

The first of said chambers is provided with an inlet for receiving the liquid waste, means for cutting off the entrance of the waste and causing it to be transferred to the second chamber, and means for supplying a dispersing agent to said first chamber.

The second chamber is in turn provided with a waste outlet, means for expelling said waste, and means for supplying a disinfecting agent to said second chamber.

The apparatus of the invention is thus subdivided into two regions: a first region for receiving the waste in which said waste is subjected to a dispersing treatment, and a second region in which it undergoes a disinfecting treatment for all types of virus, bacteria and pathogenic elements that exist, enabling the waste to be poured in a completely oxidized state into the sewage system.

Both the first phase of homogenization by means of the dispersant and the second phase of disinfection are speeded up by the action of the stirrers in both chambers.

Preferably, the two chambers of which the apparatus of the invention consists are superposed such that the first chamber is positioned above the second chamber. The stirrers of each of the two chambers are driven via coaxial axes from a common motor positioned above the first chamber.

Each of the chambers is provided with a set of syphons and overflow pipes which enable the level of liquid to be controlled and ensure that uncontrolled loss by accumulation is impossible.

### BRIEF DESCRIPTION OF THE DRAWINGS

The characteristics of the invention as defined in the claims are described below in greater detail with reference to the accompanying drawings which show by way of non-limiting example an apparatus for the treatment of liquid hospital waste according to the present invention.

In the drawings:

Figure 1 shows a vertical cross section of an apparatus constituted according to the invention.

Figure 2 is a plan view of the apparatus shown in figure 1 with the upper cover sectioned along the line II-II of figure 1.

Figure 3 is a partial vertical section of the apparatus, similar to figure 1, showing an alternative embodiment.

Figure 4 is a lateral elevation of one of the pressurestats included in the apparatus shown in figures 1 to 3.

### DESCRIPTION OF THE PREFERRED EMBODIMENTS

According to the present invention, the apparatus for the treatment of liquid hospital waste comprises a first chamber 1 and a second chamber 2 which are independent from each other and superposed, it being possible for both chambers to be circular in shape.

In the embodiment shown in the drawings, the chamber 2 is closed above by the walls of the chamber 1, which is provided with a closing cover 3 above which is arranged an enclosure 4 which, together with said cover, defines a region 5 which houses the various control mechanisms, as will seen later on. Finally, beneath the chamber 2 is arranged a hollow bed or base 6 through which the apparatus is drained.

The first chamber 1 is provided with an inlet for receiving the liquid waste, which may enter automatically via the pipe 7 or manually via the receiving funnel or mouth 8. In either case, the waste inlet pipe includes a syphon which ensures a watertight seal, thereby preventing any vapours that may be produced inside the apparatus during the treatment process from reaching the outside.

The chambers 1 and 2 are connected together by a syphon 9 and a overflow pipe 10. The liquid waste is racked from the chamber 1 to the chamber 2 by means of the syphon 9, the overflow pipe 10 acting as a safety device to prevent the waste inside the chamber 1 from exceeding a predetermined level.

The chamber 2 is turn provided with a waste outlet 11 to which is connected a pump 12 which pumps the waste into the drainage pipe 13 which can be connected to the sewage system.

The assembly described is completely watertight and is made of plastic materials which are inert to the effects of the chemicals involved in the treatment process.

If the waste enters the chamber 1 automatically, an electrically operated valve is provided to cut off the inlet to said chamber in the event of an alarm or over-saturation.

Stirrers 14 and 15 are arranged inside the chambers 1 and 2, said stirrers being joined to the corresponding coaxial axes 16 and 17 respectively, said axes being fixed to the axis of a motor 18 housed in the upper part 5 of the apparatus. Furthermore, a gauge column 19 is arranged between the axes 16 and 17, making it unnecessary to use seals, retainers or supports etc. The stirrers 14 and 15 speed up and perfectly homogenize the processes that take place in each of the chambers 1 and 2.

Two pumps 20 and 21 are further provided in the upper part 5 of the apparatus, the first of said pumps injecting disinfecting agents into the tank 2 and the pump 21 being used for injecting a dispersing agent into the tank 1.

Five pressurestats 22, 23, 24, 25, and 26 are further provided in the upper part 5 of the apparatus.

The pressurestat 22 is designed to control the entrance of the waste into the first tank 1. When the waste in this tank reaches a predetermined amount, the pressurestat 22 closes the waste inlet and activates an electrically operated valve 27 which opens the water inlet to the chamber 1 such that the level rises until the syphon 9 starts, at which point the racking from the chamber 1 to the chamber 2 begins. The pressurestat 23 ensures that the water enters the tank 1 for right length of time and, should said flow of water entering the chamber be cut off, it produces an alarm. The pressurestat 25 ensures the entrance of the right amount of dispersing agent into the first tank 1 and, as the previous case, produces an alarm if the flow of said dispersing agent is cut off.

Finally, the pressurestat 24 ensures that the second tank is evacuated and the pressurestat 26 ensures the entrance of the disinfecting agent into said second tank. Should the operation of evacuating the second tank be delayed or the entrance of disinfectant be cut off, the corresponding alarms are generated.

The pressurestat 24 activates the electrically-driven pump 12 by means of which the second chamber 2 is evacuated.

The operational process of the apparatus of the invention is as follows: as waste enters the first compartment 1 it is treated with dispersant previously injected into said compartment, the waste being homogenized by means of the stirrer 14. When the waste inside the chamber 1 reaches a predetermined level, the pressurestat 22 closes the waste inlet and activates an electrically operated valve 27, thereby allowing the entrance of the right amount of water to start the syphon 9 such that the waste is transferred to the second tank 2. Disinfectant is injected into this second tank by means of the pump 20 and, with the help of the stirrer 15, the waste is disinfected.

At the same time that the disinfectant is injected into the second tank, dispersant is injected into the first tank by means of the electrically-driven pump 21 to initiate another cycle simultaneously. If no dispersant or disinfectant is injected, the operation of the apparatus interrupted and one or several warning signals are given so that the problem can be rectified, thereby preventing the waste from being discharged before it has been properly treated.

With the constitution described fully automatic operation is obtained, from the entrance to the discharge of the waste, which is treated in the process. Furthermore, the apparatus is self-cleaning without the need for human intervention, thereby avoiding the associated sanitary risks.

The process of the apparatus of the invention terminates when the pump 12 is activated, by means of a timer for example, causing the waste to be expelled to the outside via the syphon 13. The pressurestat 24 ensures that the second tank 2 is completely evacuated.

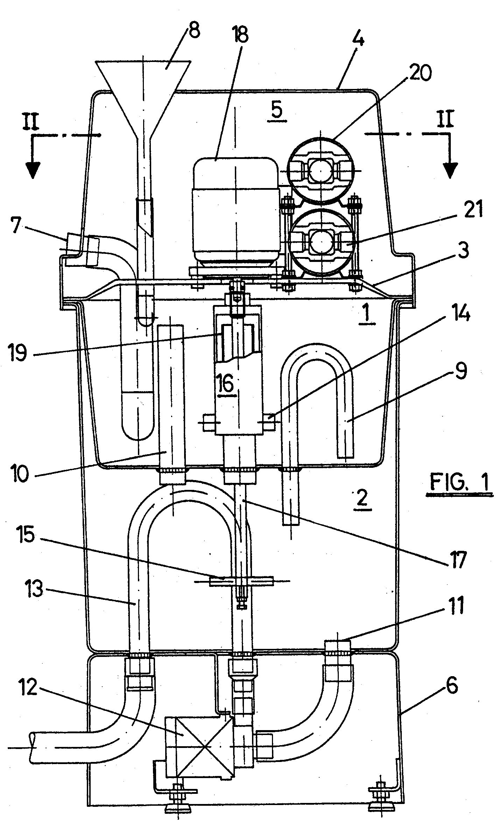

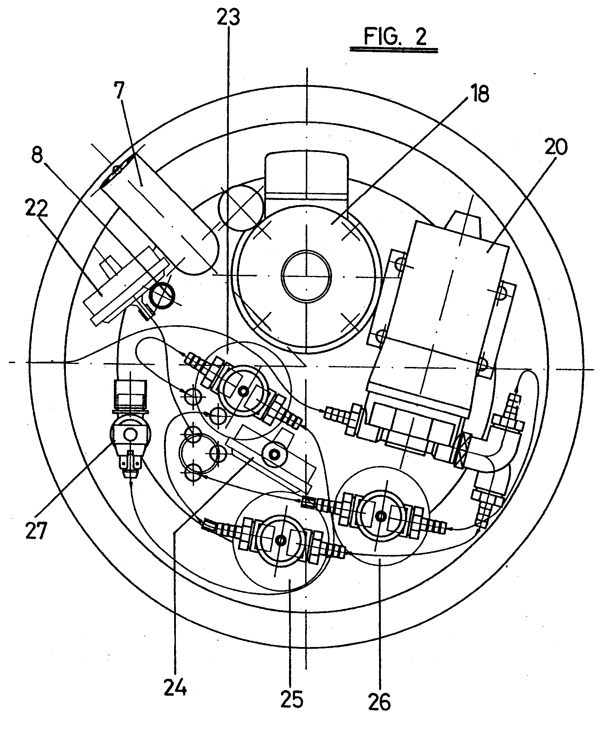

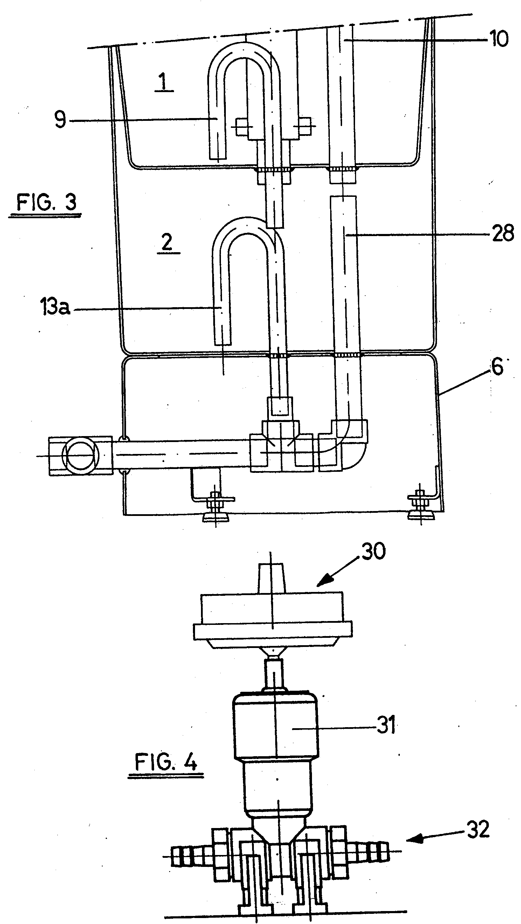

The electrically-driven pump 12 for evacuating the second tank 2 may be substituted by a syphon 13a, figure 3, which starts when the waste in the compartment 2 reaches a certain level. In this case the compartment 2 may be further provided with a safety overflow pipe 28. Otherwise, the embodiment shown in figure 3 coincides with those shown in figures 1 and 2.

The arrangement of the pressurestats enables a series of alarms to be fitted, a drain alarm for the tank 2, an alarm to indicate that no water is entering the tank 1, an alarm to indicate that no disinfectant is entering the tank 2 and an alarm to indicate that no dispersant is entering. It is further provided with an alarm in the case the stirrers malfunction.

The different pressurestats, indicated by the general reference 30 in figure 4, are constituted in such a way that they measure the variation in pressure produced inside an air chamber 31 through which runs a pipe 32 inside which flows the liquid which is introduced into each of the chambers 1 and 2 in each case.

## Claims

1. An apparatus for the treatment of liquid hospital waste, characterized in that it comprises a first and a second watertight treatment chamber, connected together by means of a syphon and an overflow pipe, and provided with stirrers for the liquid waste; said first chamber being provided with an inlet for receiving the liquid waste, means for cutting off the entrance of the waste and causing it to be transferred to the second chamber, and means for supplying a dispersing agent to said first chamber; and said second chamber being provided with a waste outlet, means for expelling said waste, and means for supplying a disinfecting agent to said second chamber.

2. An apparatus according to claim 1, characterized in that the two chambers are superposed such that the first chamber is positioned above the second chamber, the stirrers of both chambers being driven via coaxial axes from a common motor positioned above the first chamber.

3. An apparatus according to claim 1, characterized in that the means for cutting off the entrance of the waste into the first chamber and causing it to be transferred from said first chamber to the second chamber comprise a detector for detecting a predetermined maximum volume of waste in the first chamber, an electrically operated valve for supplying water to said first chamber and a device for controlling the entrance of the water; said electrically operated valve being activated by said level detector such that it opens, and remaining open until the device that controls the entrance of the water detects the priming of the syphon which connects the two chambers and the start of the transfer of the waste to the second chamber.

4. An apparatus according to claim 1, characterized in that the means of supplying a dispersing agent to the first chamber consist of a detector for detecting a predetermined minimum volume of waste in said first chamber and a pump for supplying the dispersant, said pump being activated by said detector to cause the injection of a predetermined amount of dispersant.

5. An apparatus according to claim 1, characterized in that the means of supplying a disinfecting agent to the second chamber comprise a detector for detecting a predetermined amount of waste in the second chamber and a pump which is activated by the detector to cause the injection of a predetermined volume of dispersant.

6. An apparatus according to claim 1, characterized in that the means of expelling the waste from the second chamber comprise a detector for detecting a predetermined volume of waste and a pump which is activated by said detector.

7. An apparatus according to claim 1, characterized in that the means of expelling the waste from the second chamber comprise a syphon, which starts when the volume of waste inside the chamber reaches a predetermined value, and a detector for detecting the waste and ensuring that said chamber is completely evacuated.

8. An apparatus according to claims 3 and 4, characterized in that each of said detectors consists of a pressurestat.

9. An apparatus according to claim 8, characterized in that at least one of the pressurestats which ensures the entrance of water, dispersant and disinfectant into the chambers measures the variations in pressure produced in an auxiliary air chamber through which runs a pipe inside which flows the liquid which is introduced into the chamber.
